# EUROPEAN PATENT APPLICATION

(11) **EP 1 686 178 A1**
(43) Date of publication of application: **02.08.2006**
(21) Application number: 05001036.2
(22) Date of filing: 19.01.2005
(51) Int. Cl.: C12N 15/85, C12N 5/08

(54) **Human hepatocyte-like cells and uses thereof**

(71) Applicant: Ochiya, Takahiro, Tokyo 104-0045 (JP); Effector Cell Institute, Inc., Tokyo 153-0041 (JP)
(72) Inventor: Ochiya, Takahiro, 1-1, Tsukiji, Chuo-ku, Tokyo 104-0045 (JP); Teratani, Takumi, Matsudo-shi, Chiba 271-0087 (JP)
(74) Representative: Keller, Günter

(57) **Abstract**

The present invention provides modified methods of producing human hepatocyte-like cells which exhibit phenotypes more similar to those of human hepatocytes. The present invention also provides the human hepatocyte-like cells and uses thereof. The methods of the present invention comprises a prolonged incubation period and addition of dexamethasone to the culture medium. The methods of the inventors produce human hepatocyte-like cells whose morphology is more similar to that of human hepatocytes, compared with the conventional system. The cells produced have both morphological and functional features of primary culture cells from normal human liver, including cytochrome P450 (CYP), multi-drug resistance-associated protein (MRP), and multi-drug protein (MDR). The use of human hepatocyte-like cells of the present invention enables, without using any animal model, assessment of metabolism and hepatotoxicity of test compounds, which are drug candidates , and screening for therapeutic agents for hepatic diseases, inhibitors to hepatitis virus infection, and therapeutic agents for viral hepatitis.

## Description

### FIELD OF THE INVENTION

The present invention relates to human hepatocyte-like cells and uses thereof.

### BACKGROUND OF THE INVENTION

Human mesenchymal stem cells are thought to be multipotent cells, which are present in adult bone marrow, can replicate as undifferentiated cells and have the potential to lineages of several different tissues.

Mesenchymal stem cells (MSCs) were first isolated from bone marrow (BM) by Friedenstein (1982), by simple plating on plastic in the presence of fetal calf serum (M. F. Pittenger et al., Science. 284, 143, 1999). Human MSCs (hMSCs) isolated from BM aspirates share a general immunophenotype and are uniformly positive for SH2, SH3, CD29, CD44, CD71, CD90, CD106, CD120a, CD124, but are negative for CD14, CD34, and the leukocyte common antigen CD45. hMSCs are multipotent, capable of differentiating into at least three lineages (osteogenic, chondrogenic and adipogenic) when cultured under defined conditions *in vitro* (M. F. Pittenger et al., Science. 284, 143, 1999).

Previous attempts at differentiation of mature hepatocytes from adult BM including hMSCs (CD34-positive cells fraction) have been reported (S. A. Camper, S. M. Tilghman, Biotechnology. 16, 81, 1991; J. L. Nahon, Biochimie. 69, 445, 1987; A. Medyinsky, A. Smith, Nature. 422, 823, 2003). None, however, has induced functional hepatocytes by direct differentiation *in vitro*.

### SUMMARY OF THE INVENTION

Recently, the present inventors have identified growth factors that allow direct hepatic fate-specification from mouse, rat, and monkey embryonic stem (ES) cells using simple adherent monoculture conditions. The hepatic induction factor cocktail (HIFC) differentiation system comprises three steps and is highly efficient with approximately 3.0x 10⁶ functional mature hepatocytes produced when 1.0x 10⁵ undifferentiated mouse ES cells were used as starting material. The ES cell-derived hepatocytes revealed the characteristics of mature hepatocytes with respect to the expression of hepatocyte-specific genes by RT-PCR and several metabolic activities; furthermore, they exhibited transplantable potential in animals.

In this context, an objective of the present invention is to produce human hepatocyte-like cells which exhibit phenotypes more similar to those of human hepatocytes using a modification of the conventional system. Another objective is to provide human hepatocyte-like cells and uses thereof.

Through trial and error, the present inventors revealed that human hepatocyte-like cells whose morphology was more similar to that of human hepatocytes could be produced by modifying the conventional system to prolong the incubation period and add dexamethasone to the culture medium. It was also found that the cells had both morphological and functional features of primary culture cells from normal human liver, containing cytochrome P450 (CYP), multi-drug resistance-associated protein (MRP), and multi-drug protein (MDR).

The use of human hepatocyte-like cells of the present invention enables without using any animal model, assessment of metabolism and hepatotoxicity of test compounds, which are drug candidates, and screening for therapeutic agents for hepatic diseases, inhibitors to hepatitis virus infection, and therapeutic agents for viral hepatitis.

Specifically, the present invention provides:
(1) A method for preparing human hepatocyte-like cells, which comprises the steps of: (a) incubating undifferentiated cells derived from human in a culture medium containing a combination of growth factors selected from (i) to (iii) below:
   (i) acidic fibroblast growth factor, fibroblast growth factor 4, and hepatocyte growth factor;
   (ii) acidic fibroblast growth factor and a growth factor selected from the group consisting of activin A, epidermal growth factor, and β-nerve growth factor; and
   (iii) fibroblast growth factor 4 and a growth factor selected from the group consisting of activin A and hepatocyte growth factor; ;
      (b) incubating the cells cultured in step (a) in a culture medium containing oncostatin M; and
      (c) incubating the cells cultured in step (b) in a culture medium containing dexamethasone, wherein the total culturing period is about 2 to about 13 weeks;
(2) The method according to (1), which comprises using a collagen-coated culture dish;
(3) The method according to (1), wherein the undifferentiated cells derived from human are embryonic stem cells, adult stem cells, mesenchymal stem cells, cord blood cells, or somatic cells with artificially conferred multipotency;
(4) Human hepatocyte-like cells prepared by the method according to any one of (1) to (3);
(5) A therapeutic agent for hepatic diseases, which comprises the human hepatocyte-like cells according to (4);
(6) A method for assessing the metabolism of a test compound, which comprises the steps of:
   (a) contacting a test compound with the human hepatocyte-like cells according to (4); and
   (b) determining the metabolism of the test compound contacted with the human hepatocyte-like cells;
(7) A method for assessing hepatotoxicity of a test compound, which comprises the steps of:
   (a) contacting a test compound with the human hepatocyte-like cells according to (4); and
   (b) determining the degree of damage of the human hepatocyte-like cells contacted with the test compound;
(8) A method of screening for a therapeutic agent for hepatic diseases, which comprises the steps of:
   (a) contacting a test compound with the human hepatocyte-like cells according to (4);
   (b) determining the function of the human hepatocyte-like cells contacted with the test compound; and
   (c) selecting a compound that enhances the function of the human hepatocyte-like cells contacted with the test compound;
(9) A method of screening for an inhibitor to hepatitis virus infection, which comprises the steps of:
   (a) contacting the human hepatocyte-like cells according to (4) with hepatitis virus in the presence of a test compound;
   (b) examining the infection of hepatitis virus to the human hepatocyte-like cells contacted with hepatitis virus; and
   (c) selecting a compound that inhibits the infection of hepatitis virus; and
(10) A method of screening for a therapeutic agent for viral hepatitis, which comprises the steps of:
   (a) contacting the human hepatocyte-like cells according to (4) with hepatitis virus;
   (b) contacting a test compound with the human hepatocyte-like cells infected with hepatitis virus;
   (c) determining the degree of hepatitis virus propagationin the cells contacted with the test compound; and
   (d) selecting a compound that inhibits the propagation of hepatitis virus.

The words "a", "an", and "the" as used herein mean "at least one" unless otherwise specifically indicated.

### BRIEF DESCRIPTION OF THE DRAWINGS

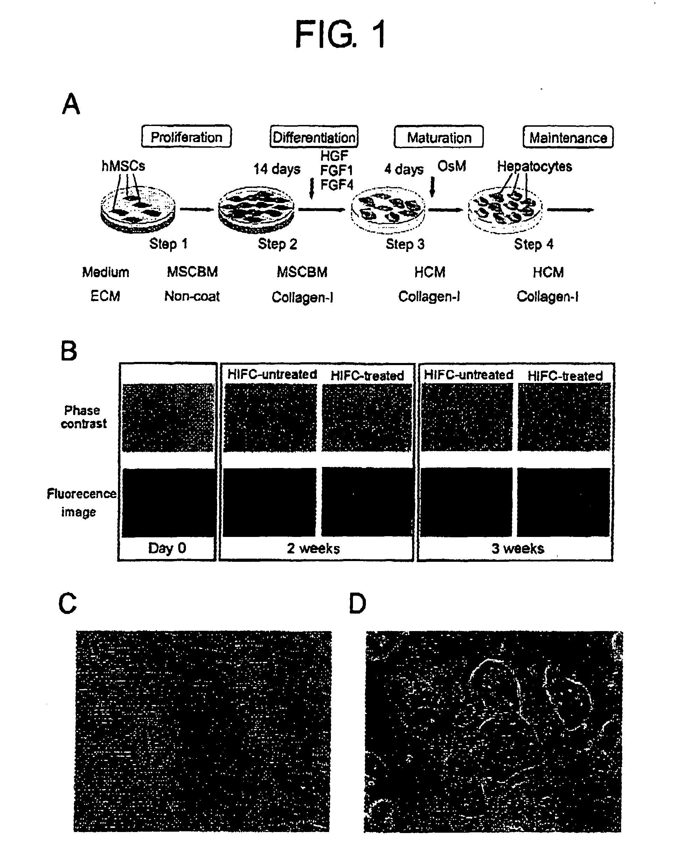

Fig. 1 shows the hepatic differentiation of hMSCs in an adherent monoculture.
   A: an integrated schematic representation of the differentiation protocol for the induction of hepatocytes from hMSCs in monolayer culture (steps 1 to 4).
   B: photographs showing morphological changes and induction of GFP expression during the course of hepatocyte differentiation from pALB-EGFP/hMSCs.
   C: a photograph showing HIFC-untreated hMSCs.
   D: a photograph showing HIFC-treated hMSCs.

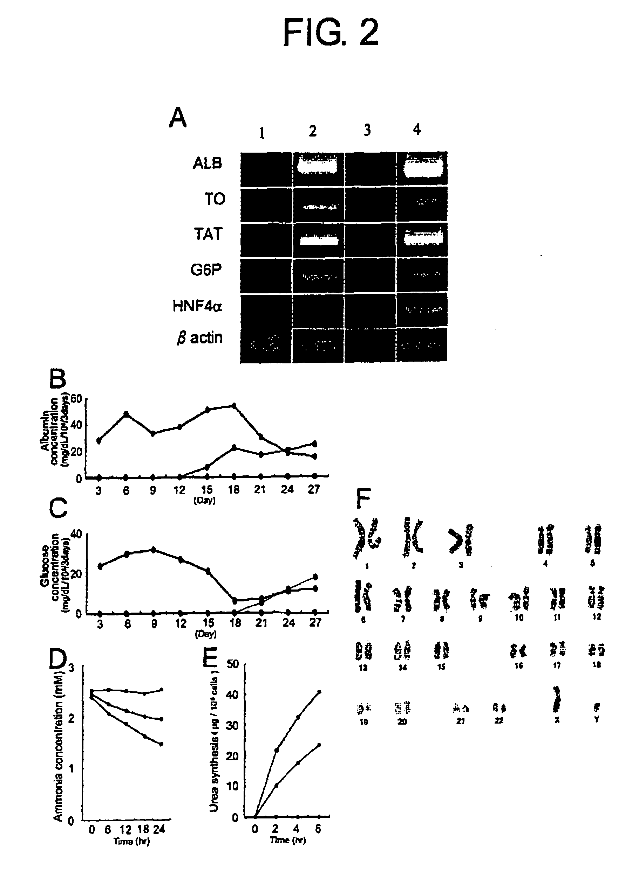

Fig.2 shows the expression of liver-specific markers and *in vitro* functions of hMSC-derived hepatocytes.
   A: photographs showing hepatocyte-specific gene expression by RT-PCR analysis. RNA was isolated from HIFC-untreated hMSCs (lane 1), HIFC-stimulated hMSCs (lane 2), and no-template (lane 3, negative control) and cDNA of human cultured hepatocytes (lane 4, positive control; AFP is HepG2 cells cDNA).
   B: albumin production;
   C: glucose production;
   D: ammonia elimination;
   E: urea synthesis.
      In the graphs B-E (1), (2), and (3) indicate GFP-positive hMSCs, HIFC-untreated hMSCs, and human cultured hepatocytes, respectively. Data are reported as the mean ± s.e.m. and analyzed using ANOVA. The number of experiments was three in each experimental group.
   F: a photograph showing G-banded karyotype of HIFC-stimulated hMSCs.

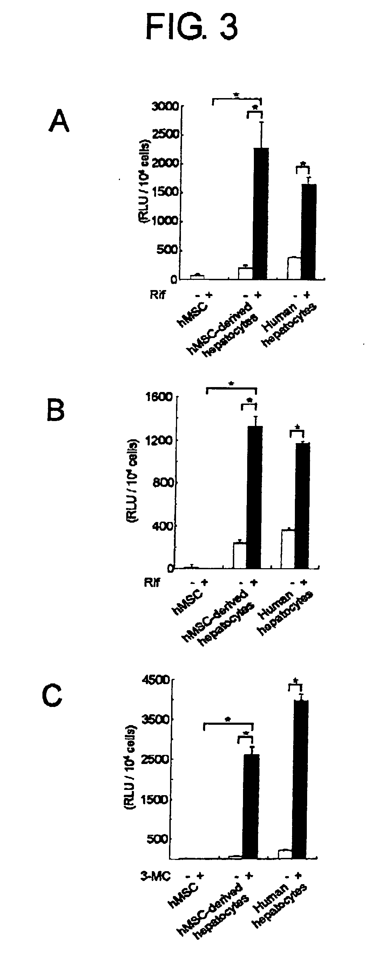

Fig.3 shows cytochrome P450 activities in hMSC-derived hepatocytes. P450 activities for CYP3A4 (A), CYP2C9 (B) and CYP1A1 (C) were measured in hMSC-derived hepatocytes, human primary culture hepatocytes (positive control), HIFC-untreated hMSC (negative control). CYP3A4 and CYP2C9 were induced by rifampicin (Rif). CYP1A1 was induced by 3-methylcholanthrene (3-MC). Raw data were normalized to mean cell volume to compensate for cell number differences. Results represent the mean ± s. e. m. (n = 3) *, P>0.05.

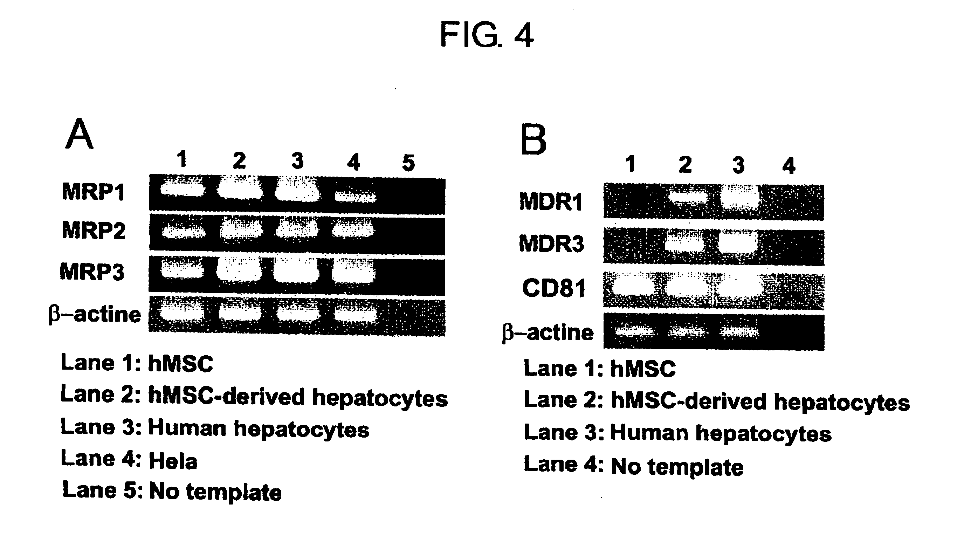

Figure 4 shows the expression patterns of MRP and MDR genes and their mRNA levels in hMSC-derived hepatocytes.
   A: a photograph showing the expression of MRP genes (MRP1-3) analyzed by RT-PCR. Gene expression patterns of: unstimulated hMSC (lane 1), HIFC-stimulated hMSC (lane 2); primary culture cells from normal human liver (lane 3; positive control); HeLa cells (lane 4); and no template (lane 5; negative control).
   B: a photograph showing the expression of MRP genes (MRP1 and 3) and CD81 analyzed by RT-PCR. Gene expression patterns of: unstimulated hMSC (lane 1), HIFC-stimulated hMSC (lane 2); primary culture cells from normal human liver (lane 3; positive control); and no template (lane 4; negative control).

### DETAILED DESCRIPTION OF THE INVENTION

The present invention provides methods for preparing human hepatocyte-like cells from undifferentiated cells derived from human.

In the methods of the present invention, first, undifferentiated cells derived from human are differentiated into human hepatocyte-like cells.

The differentiation of undifferentiated cells derived from human to human hepatocyte-like cells can be induced by culturing the undifferentiated cells derived from human in a culture medium containing a combination of growth factors selected from any one of (i) to (iii) (step (a): corresponding to step 2 described in Example):
(i) acidic fibroblast growth factor (aFGF), fibroblast growth factor 4 (FGF4), and hepatocyte growth factor (HGF);
(ii) aFGF and a growth factor selected from the group consisting of activin A, epidermal growth factor (EGF), and β-nerve growth factor (βNGF); and
(iii) FGF4 and a growth factor selected from the group consisting of activin A and HGF; and.

In the present invention, the differentiation of undifferentiated cells derived from human can be achieved more effectively by using the growth factors of (i). Herein, acidic fibroblast growth factor (aFGF) is sometimes referred to as fibroblast growth factor 1 (FGF1).

Even when the growth factors used in the present invention are not derived from human, it can induce the differentiation of undifferentiated cells derived from human.

The methods of the present invention comprises, as the next step, incubating the cultured cells of step (a) in a culture medium containing oncostatin, M (step (b): corresponding to step 3 described in Example). In this step, the differentiated cells are matured.

The methods of the present invention comprises, as the next step, incubating the cultured cells of step (b) in a culture medium containing dexamethasone (DEX) (step (c): corresponding to step 4 described in Example). More specifically, DEX is added to the culture medium after the incubation of step (b), and then the culturing is further continued. Alternatively, in the present invention, DEX may be added to the culture media in both steps (a) and (b).

In the present invention, the undifferentiated cells derived from human include, for example, embryonic stem cells (ES cells), adult stem cells, mesenchymal stem cells, cord blood cells, and cells with artificially conferred multipotency. However, any cell can be used as cells with artificially conferred multipotency as long as it can be differentiated into various cell types. The "cells with artificially conferred multipotency" includes somatic cells with multipotency conferred by cloning techniques such as gene manipulation.

When mesenchymal stem cells are used in the methods of the present invention, the cells may be pre-cultured as conducted in Example (corresponding to step 1 described in Example) before the induction of differentiation. Alternatively the pre-culturing step may be omitted. In the pre-culturing step, undifferentiated mesenchymal stem cells can be proliferated with maintaining their multipotency.

Alternatively, when ES cells are used in the methods of the present invention, the cells may be pre-cultured in a culture medium containing at least one growth factor selected from the group consisting of retinoic acid (RA), leukemia inhibitory factor (LIF), and HGF before step (a). With this pre-culturing step, the differentiation of ES cells can be induced efficiently. When the cells are pre-cultured in a culture medium containing LIF and/or HGF in addition to RA, the differentiation of ES cells can be induced more efficient.

The cell culturing methods used in the present invention include: a two-dimensional culturing method using culture dishes coated with different matrices or using culture dishes coated or not coated with a matrix; a three-dimensional culturing method using soft gel, such as matrigel, or using collagen sponge or such; and a culturing method using them in combination. A preferred method is the two-dimensional culturing method using culture dishes coated with different matrices or using culture dishes coated or not coated with a matrix.

When mesenchymal stem cells are used in the methods of the present invention, the cells can be cultured in non-coated culture dishes in the pre-culturing step and in collagen-coated culture dishes (preferably, in type-I collagen-coated culture dishes; hereinafter the same) in steps (a), (b), and (c). Gelatin-coated culture dishes may be used instead of collagen-coated culture dishes in step (a), and laminin-, atelocollagen-, or hyaluronic acid-coated culture dishes may be used instead of collagen-coated culture dishes in steps (b) and (c).

When ES cells are used in the methods of the present invention, the cells can be cultured in gelatin-coated culture dishes in the pre-culturing step and step (a), in collagen-coated culture dishes or laminin-coated culture dishes in step (b), and in collagen-coated culture dishes, laminin-coated culture dishes, atelocollagen-coated culture dishes, or hyaluronic acid-coated culture dishes in step (c).

When other undifferentiated cells other than mesenchymal stem cells and ES cells derived from human are used in the methods of the present invention, the cells can be cultured in appropriate culture dishes selected with reference to cases of mesenchymal stem cells and ES cells.

Herein in Example, the culturing conditions of steps (a), (b), and (c), and pre-culturing step are described in more detail. The culturing conditions to be used in the methods of the present invention are not restricted to the conditions described in Example. Generally acceptable culturing conditions can also be used in the present invention. For example, the number of cells may range from 5.0x 10³ to 5.0x 10⁶ cells/culture dish at the start of induction of differentiation. There is no particular limitation on the culturing period in the methods of the present invention.

When mesenchymal stem cells are used in the methods of the present invention, the overall period of incubation throughout steps (a), (b), and (c) is, but is not limited to, preferably about 2 to about 13 weeks, more preferably about 3 to about 12 weeks, still more preferably about 3 to about 10 weeks, yet more preferably about 3 to about 8 weeks.

Furthermore, when mesenchymal stem cells are used in the methods of the present invention, combinations of each incubation period of steps (a), (b), and (c) are, without limitation, exemplified below:
1) step (a): 10 to 12 days, step (b): 2 to 3 days, step (c): 0 day;
2) step (a): 12 to 16 days, step (b): 4 to 7 days, step (c): 1 day to 5 days;
3) step (a): 14 to 21 days, step (b): 4 to 7 days, step (c): 3 to 30 days;
4) step (a): 18 to 24 days, step (b): 7 to 10 days, step (c): 25 to 35 days; and
5) step (a): 25 to 28 days, step (b): 11 to 14 days, step (c): 36 to 46 days.

The incubation period for the pre-culturing step is, for example, but is not limited to, about 4 days to about 6 days.

When undifferentiated cells other than mesenchymal stem cells derived from human are used in the methods of the present invention, the overall period of incubation throughout steps (a), (b), and (c), each incubation period of steps (a), (b), and (c), and the incubation period of the pre-culturing step can be appropriately decided with reference to the incubation period in case of mesenchymal stem cells.

The growth factor to be used in the present invention includes, for example, but is not limited to, RA (all-trans-Retinoic Acid: Wako Pure Chemical Industries, Ltd.), LIF (ESGRO^{™} (10⁷ units): Funakoshi Co. Ltd.), HGF (Human HGF: VERITAS), aFGF (Human FGF-acidic: VERITAS), FGF4 (Human FGF-4: VERITAS), OsM (Human Oncostatin M: VERITAS), and DEX (Dexamethasone: Sanko Junyaku).

According to the present invention, human hepatocyte-like cells, in particular mature human hepatocyte-like cells, can be prepared by the differentiation-induction method described above.

The differentiated cells can be confirmed to be human hepatocyte-like cells by using a hepatocyte marker or a hepatocyte function as an indicator.

The hepatocyte marker includes ALB, TTR, CYP3A4, CYP1A1, MRP1, MRP2, MRP3, MDR1, and MDR3. The hepatocyte function includes, for example, the ability of producing glucose, the ability of metabolizing ammonia, the ability of producing albumin, and the ability of synthesizing urea. The presence of ability of producing glucose can confirmed by analyzing the culture supernatant for the glucose level using the glucose oxidase method. The presence of ability of metabolizing ammonia can be confirmed by analyzing the culture medium for the ammonia level using a modified indophenol method (Horn DB & Squire CR, Chim. Acta. 14, 185-194, 1966). The presence of ability of producing albumin can be confirmed by analyzing the culture media for the albumin concentration using an assay method of determining serum albumin concentrations. The presence of ability of synthesizing urea can be confirmed by using for example Colorimetric assay (Sigma)..

The present invention also provides human hepatocyte-like cells prepared by the methods of the present invention. As compared to human hepatocyte-like cells prepared by the conventional method, human hepatocyte-like cells prepared by the methods of the present invention are more similar in function and morphology to mature human hepatocytes. Thus, the human hepatocyte-like cells of the present invention are useful, for example, in the medical field (for example, in the field of regeneration medicine).

For example, hepatic diseases can be treated by using human hepatocyte-like cells of the present invention. For example, hepatic diseases can be treated by a method of transplanting the human hepatocyte-like cells directly through the hepatic portal vein or after being embedded in collagen, polyurethane, or any other known biocompatible material. Thus, the present invention also provides the use of the human hepatocyte-like cells prepared by the methods described above. More specifically, the present invention provides therapeutic agents for hepatic diseases, which comprise the human hepatocyte-like cells. In addition, the present invention also provides therapeutic methods for hepatic diseases, which use the human hepatocyte-like cells. The hepatic diseases to be treated by the present invention include, but are not limited to, hepatic cirrhosis, fulminant hepatitis, biliary atresia, hepatic carcinoma, and hepatitis (for example, viral hepatitis and alcoholic hepatitis).

Furthermore, the human hepatocyte-like cells of the present invention are also useful, for example, in the field of clinical study to aim at treating hepatic diseases. For example, the human hepatocyte-like cells of the present invention can be used in studies to develop artificial organs (artificial liver or such). Furthermore, the human hepatocyte-like cells of the present invention are useful in the field involved in the development of pharmaceuticals and foods, as described below. Specifically, the cells can be used to assess test compounds for their metabolism and hepatotoxicity and to screen for therapeutic agents for hepatic diseases, inhibitors to hepatitis virus infection, or therapeutic agents for viral hepatitis.

Test compounds can be assessed for their metabolism and hepatotoxicity by using human hepatocyte-like cells prepared by the methods of the present invention.

Animal models or such have been employed previously to assess test compounds for their metabolism and hepatotoxicity. However, there has been limitation on the number of test compounds treated at a time, and another problem is that results obtained through the assessment of test compounds using animal models or such are not directly applicable to human cases. Thus, methods that comprise using human hepatocarcinoma cell lines or primary culture cells from normal human liver have been used to assess test compounds for the purposes described above. However, assessment results obtained using human hepatocarcinoma cell lines are not always applicable to human normal hepatocytes. Because human hepatocarcinoma cell lines are cancer cells. On the other hand, primary culture cells from normal human liver have problems on the cost and stable supply. Cell lines obtained by immortalizing primary culture cells from normal human liver have been reported to have lower CYP3A4activities as compared to unimmortalized cells (Akiyama I. et al., International Journal of Molecular Medicine, 14, 663-668,2004). Such problems can be solved by using the human hepatocyte-like cells prepared by the methods of the present invention.

The present invention provides methods for assessing test compounds for their metabolism. In the methods, a test compound is contacted with the human hepatocyte-like cells prepared by the methods of the present invention. Then, the metabolism of the test compound contacted with the human hepatocyte-like cell is determined.

There is no particular limitation on the type of test compounds to be used in the present invention. Test compounds include, for example, but are not limited to, single compounds, such as xenobiotics, natural compounds, organic compounds, inorganic compounds, proteins, and peptides; compound libraries; expression products of gene libraries; cell extracts; cell culture supematants; products of fermentation microorganisms; extracts of marine organisms; and plant extracts. Exemplary xenobiotics include, for example, but are not limited to, candidate compounds for drugs and foods, and known drugs and foods. Any xenobiotic can be used in the present invention as long as it is a material foreign to the living body. More specifically, the xenobiotics include, for example, rifampin, dexamethasone, phenobarbital, ciglirazone, phenytoin, efavirenz, simvastatin, β-naphthoflavone, omeprazoie, clotrimazole, and 3-methylcholanthrene.

The "contact" used in the methods of the present invention can be achieved typically by adding a test compound to a culture medium or culture solution, but is not limited thereto. When a test compound is a protein, the "contact" can be achieved by introducing a DNA vector expressing the protein into cells.

The metabolism of a test compound can be determined by a method known to those skilled in the art. For example, when a metabolic product of the test compound is detected, the test compound is judged to have been metabolized. Alternatively, when the gene encoding an enzyme, such as CYP (cytochrome p450), MDR, or MPR, is expressed or the activity of such an enzyme is increased after the cell is contacted with the test compound, the test compound is judged to have been metabolized.

The present invention also provides methods for assessing test compounds for their hepatotoxicity. In the method, a test compound is contacted with the human hepatocyte-like cells prepared by the methods of the present invention. Then the degree of damage of the human hepatocyte-like cells contacted with the test compounds is determined. The degree of damage can be determined, for example, by using the viability of the human hepatocyte-like cells or a liver function marker, such as GOT and GPT, as an indicator.

For example, when the viability of the human hepatocyte-like cells is decreased after addition of a test compound to culture medium of the human hepatocyte-like cells, the test compound is judged to have hepatotoxicity. When no significant changes are detected in the viability, the test compound is judged to have no hepatotoxicity. Alternatively, for example, when the level of GOT or GPT is increased in the culture medium of the human hepatocyte-like cells after addition of the test compound to the culture medium, the test compound is judged to have hepatotoxicity. When no significant changes are detected in the level of GOT or GPT, the test compound is judged to have no hepatotoxicity.

The hepatotoxicity of the test compound can be assessed more reliably by using control compounds which have hepatotoxicity or no hepatotoxicity.

In addition, the present invention provides methods of screening for therapeutic agents for hepatic diseases. In the method, a test compound is contacted with human hepatocyte-like cells prepared by the methods of the present invention. Then, the function of human hepatocyte-like cells contacted with the test compound is determined to thereby select a compound that enhances the function of the human hepatocyte-like cells contacted with the test compound.

The function of hepatocyte-like cells according to the present invention can be assessed by using as an indicator the ability of producing glucose, the ability of metabolizing ammonia, the ability of producing albumin, the ability of synthesizing urea, or the activity of an enzyme, such as CYP.

The presence of ability of producing glucose can be confirmed by analyzing the culture supernatant for the glucose level using the glucose oxidase method. The presence of ability of metabolizing ammonia can be confirmed by analyzing the culture medium for the ammonia level by the modified indophenol method (Horn DB & Squire CR, Chim. Acta. 14, 185-194, 1966). There is no particular limitation on the type of CYP of the present invention. The presence of ability of producing albumin can be confirmed by subjecting the culture media to a known method of determining serum albumin concentrations. The presence of ability of synthesizing urea can be confirmed by using for example Colorimetric assay (Sigma). The CYP includes, for example, CYP1A1, CYP2C8, CYP2C9, and CYP3A4. The activity of CYP can be determined by a method known to those skilled in the art.

The human hepatocyte-like cells prepared by the methods of the present invention can be infected with hepatitis virus, because the cells are more similar to mature human hepatocytes in function and morphology.

The present invention provides a method of screening for inhibitors to hepatitis virus infection. In the method, hepatitis virus is contacted with human hepatocyte-like cells prepared by the methods of the present invention in the presence of a test compound. Then the infection of hepatitis virus to the human hepatocyte-like cells contacted with hepatitis virus is determined to thereby select a compound inhibiting the infection of hepatitis virus. The contact of the cells with hepatitis virus can be achieved by a conventional method.

There is no particular limitation on the type of hepatitis virus. The virus includes hepatitis C virus, hepatitis A virus, and hepatitis B virus. These hepatitis viruses may be viral strains established or viruses isolated directly from subjects infected with hepatitis virus. The hepatitis viruses may be purified or crude samples (for example, in sera obtained from subjects infected with hepatitis virus).

So far no high efficient *in-vitro* hepatitis C virus infection system has been established. It is not realistic to use hepatocytes for developing inhibitors to the infection of hepatitis C virus or therapeutic agents for hepatitis C. In addition, the life cycle of hepatitis C virus still remains unclear. Nonetheless, the present inventors have revealed that hepatitis C virus is infectious to the human hepatocyte-like cells of the present invention and the infection efficiency is exceedingly high. This suggests that the human hepatocyte-like cells of the present invention can be used to screen for inhibitors to hepatitis C virus infection and therapeutic agents for hepatitis C, and to elucidate the life cycle of hepatitis C.

The infection of hepatitis virus can be tested, for example, by determining the amount of hepatitis virus in a cell as an indicator. The amount of hepatitis virus in a cell can be determined, for example, based on the amount of RNA of hepatitis virus in a cell as an indicator. The amount of RNA of hepatitis virus can be determined by a conventional method. The amount may be determined using the method described inT. Takeuch. et al., Real-Time Detection System for Quantification of Hepatitis C Virus Genome. Gastroenterology 116, 636-642, 1999).

In addition, the present invention also provides methods of screening for therapeutic agents for viral hepatitis. In the methods, hepatitis virus is contacted with human hepatocyte-like cells prepared by the methods of the present invention. Then, a test compound is contacted with the human hepatocyte-like cells infected with hepatitis virus. As the next step, the propagation of hepatitis virus in the cells contacted with the test compound is determined, thereby selecting a compound that inhibits the propagation of hepatitis virus.

The thus-selected compounds of the present invention, which inhibit the propagation of hepatitis virus, include: (1) compounds that inhibit the propagation of hepatitis virus in the cells as compared to the cells with which test compounds have not been contacted; (2) compounds that completely inhibit the propagation of hepatitis virus; and (3) compounds that completely eliminate hepatitis virus particles. The propagation or elimination of hepatitis virus can be tested by determining the amount of hepatitis virus particles in a cell.

The methods of the present invention can be conducted at the cell level and are useful as a tool to elucidate differentiation mechanisms to hepatocytes at the molecular level. The methods can be substituted, as a new assay system, for conventional animal tests for carcinogenicity or safety assessment of food additives or anti-cancer drugs. Furthermore, the methods of the present invention is applicable to the development of artificial organs. For example, toxicity evaluation, food safety assessment, and such tests are mainly performed using rats. However, the number of test compounds tested at a time is limited because of a limited test space. Furthermore, the assessment results obtained using rats are not directly applicable to human cases, because human and rat arc considerably different animal species. Thus, human cultured cells are being substituted for animals in evaluation tests. The methods of the present invention can provide hepatocytes stably at a relatively low cost. The human hepatocyte-like cells obtained by the methods of the present invention can be used for studying methods of preventing and treating hepatic viral infection. Especially, since hepatitis C virus infects only human hepatocytes, it has been difficult to obtain tools for studying methods of preventing and treating hepatitis C virus infection. The present invention would make such studies possible by using an *in vitro* system, namely at the cell level. Furthermore, the methods of the present invention may be used, in combination with techniques actually used in dialysis treatment and artificial heart-lung machine, in excretion blood waste products by, for example, a method of extracorporeal circulation using a container having a low antigenicity permeable membrane and filled with human hepatocyte-like cells prepared by the methods of the present invention. The present invention provides for the first time a method utilizing the induction of differentiation of ES cells or mesenchymal stem cells derived from bone marrow cells, and thus will not only serve social needs but also make immeasurable contributions to and offer great hope for regeneration medicine business. The present invention may also adequately contribute to other industries.

The present invention is illustrated in detail below with reference to Examples, but is not to be construed as being limited thereto. In the present invention, statistical analyses were carried out using a commercially available software package (StatView, SAS Institute Inc.). The student's t-test was performed for statistical evaluation, with p<0.05 considered significant. When more than 2 samples were compared, the one-way ANOVA test was performed and data are presented as the mean ± s.e.m. *All in vitro* results were derived from at least three independent experiments.

### EXAMPLE 1

### Culturing human mesenchymal stem cells and induction of their differentiation

The human mesenchymal stem cell (hMSC) line was obtained from TaKaRa (Japan) and cultured in DMEM containing 5% fetal bovine serum under 5% CO₂ in a humidified atmosphere at 37°C. Specificity of the mouse albumin promoter/enhancer construct (pALB-EGFP) was evaluated by green fluorescent protein (GFP) fluorescence activity (G. Quinn, T. Ochiya, M. Terada, T. Yoshida, Biochem. Biophys. Res. Commun. 276,1089,2000; C. H. Sellem, M. Frain, T. Erdos, J. M. Sala-Trapat, Dev. Biol. 102, 51, 1984). For transgene contrast, pALB-EGFP linearized plasmid DNA was used to electroporate hMSCs 48 hr following plating (420 V, 25µF, pALB-EGFP vector of 50 µg) for co. G418-resistant pALB-EGFP/hMSCs were prepared and cultured on a plastic dish with mesenchymal stem cell basal medium (MSCGM, adding 10% fetal bovine serum).

To induce maximum differentiation of hepatocytes from hMSCs, the following four steps were performed (Fig. 1A).

### Step 1: propagation of hMSC in MSCGM

pALB-EGFP/hMSCs were cultured on plastic dishes for 5 days with an hMSC culture medium.

### Step 2: HIFC treatment against pALB-EGFP/hMSC for 14 days

After 5 days the cells were passaged and cultured for 2 weeks in the presence of HIFC (FGF1, 500 ng/ml; FGF4, 40 ng/ml; HGF, 250 ng/ml; VERITAS, Tokyo, Japan) at 10⁶ cells per 100-mm type I collagen-coated plate (Asahi Techno Glass) with a hepatocyte culture medium (HCM), modified William E medium containing transferrin (5 µg/ml), hydmcortisoae-21-hemisuccinate (10⁻⁶M), Dexamethasone (10⁻⁸M), bovine serum albumin (0.5 mg/ml), ascorbic acid (2 mM), insulin (5 µg/ml) and Gentamicin (50 µg/ml) (Sanko Junyaku, Tokyo, Japan).

### Step 3: OsM treatment for four days

Cells were then washed three times with cold PBS (-) and incubated for 20 min at 37°C in PBS containing 0.05% collagenase (GIBCO-BRL, Tokyo, Japan) and 1000units/ml dispase (Godoshusei, Tokyo, Japan). The dissociated cells were washed twice with serum-free DMEM and then resuspended in HCM medium (adding transferrin, hydrocortisone hemisuccinate, bovine serum albumin, ascorbic acid, insulin, gentamicin to William E medium) containing OsM (30 ng/ml) at 10⁶ cells per 100-mm type I collagen-coated plate.

### Step 4: supply of HCM containing dexamethasone (10⁻⁸M)

Four days after plating, the cells were fed with HCM containing dexamethasone (10⁻⁸M) and incubated for three days.

The present inventors have found that even incubation period: step 2, 21 days; step 3, 7 days; step 4; 30 days; and such could obtain results described below.

GFP gene expression was monitored by fluorescence microscopy. The matrices: laminin, vitronectin and fibronectin were used at 10 µg/ml, 6 µg/ml and 1 µg/ml, respectively (Asahi Techno Glass).

The present inventors discovered that cells treated with an HIFC produced a dramatic rate of GFP-positive cells (73.2 ± 5.7%). On the other hand, no GFP positive cells appeared in cells treated with the HIFC-negative medium (Fig. 1B). Microscopic analysis of HIFC-treated hMSCs revealed a hepatocyte-like morphology with binucleate cells and bile canaliculi frequently observed after 8 weeks from start of the incubation described in step 2 (Fig. 1, C and D). These results demonstrate that the differentiation system is highly efficient with GFP-positive hepatocyte-like cells produced from undifferentiated hMSCs in culture by growth factor stimulation.

### EXAMPLE 2

### RT-PCR analysis

To clarify the characteristic features of the GFP-positive cells, the present inventors analyzed the gene expression of a variety of hepatocyte markers and liver-enriched transcription factor by RT-PCR analysis (Fig. 2A).

At first an aliquot of total RNA isolated from undifferentiated ES cells and GFP-positive cells using ISOGEN solution (Nippon Gene, Tokyo, Japan) was treated with DNase I (amplification grade; TaKaRa, Kyoto, Japan) according to the manufacturer's guidelines.

RT-PCR reactions were performed using a One-Step RT-PCR kit (QIAGEN, Tokyo, Japan).

Expression of albumin (ALB), the most abundant protein synthesized by mature hepatocytes, starts in early fetal hepatocytes (E12) and reaches a maximal level in adult hepatocytes (C. J. Pan, J. K. Lei, H. Chen, J. M. Ward, J. Y. Chou, Arch. Biochem. Biophys. 358, 17,1998). Glucose-6-phoshatase (G6P), tyrosine aminotransferase (TAT) and tryptophan 2,3-dioxygenase (TO) are definitive enzymatic markers for peri- or postnatal hepatocyte-specific differentiation (O. Greengard, Science. 163, 891, 1969; M. Nagao, T. Nakamura, A. Ichihara, Biochem. Biophys. Acta. 867, 179,1986; T. Thomas, B. R. Southwell, G. Schreber, A. Jaworowski, Placenta. 11, 413, 1990). HNF4α is essential for morphological and functional differentiation of hepatocytes, accumulation of hepatic glycogen stores and generation of a hepatic epithelium (F. Parviz et al., Nat. Genet. 34, 292, 2003).

All cDNA fragments were amplified as a single band and the marker's identity was confirmed by sequencing (Fig. 2A). This expression pattern is similar to that of primary hepatocytes. Furthermore, HIFC-treated hMSCs expressed all hepatic marker genes analyzed, whereas no hepatic markers were detectable in HIFC-untreated hMSCs.

### EXAMPLE 3

### Biochemical analyses

To further elucidate whether GFP-positive cells display hepatocyte-specific functions, biochemical analyses were performed.

One day after plating at 2x 10⁵ cells/60-mm dish, GFP-positive hepatocytes or control ES cells and normal mouse hepatocytyes were analyzed for glucose levels in the culture supernatant by the glucose oxidase method, as described previously (H. Yamamoto et al., Hepatology 37, 983, 2003; Fig. 2C). To examine the cellular activity of ammonia detoxification, GFP-positive cells or control ES cells and normal mouse hepatocytes were cultured at 2x 10⁵ cells/60-mm dish in 1.0 ml of DMEM containing 2.5 mM NH₄Cl and further incubated for 24 hours. The culture media were tested for concentrations of NH₄Cl at 0, 6, 12 and 24 hours by Ammonia-Test Wako (Wako Pure Chemicals, Tokyo, Japan) (Fig. 2D). To assay the urea synthesis ability, cells were cultured with HBSS in the presence of 5 mmol/L NH₄Cl. The medium was harvested after incubation for 0, 2, 4 and 6 hr and assayed according to the manufacturer's guidelines(Fig. 2E).

These results indicate that cultured GFP-positive cells display albumin- and glucose-producing ability and capacity to clear ammonia from the culture media and also urea synthesis ability (Fig. 2, B to E). Levels of albumin (at day 21 from the start of the incubation described in step 2), glucose (at day 21 from the start of the incubation described in step 2) and urea produced by GFP-positive cells were similar to those in monolayer cultures of primary human hepatocytes. On the other hand, HIFC-untreated hMSCs did not produce albumin, glucose or urea, and displayed no capacity to clear ammonia from the supernatant (Fig. 2, B to E). G-banding revealed that the chromosome number of GFP-positive cells was 46 and no gross chromosomal changes were observed (Fig. 2F). Thus, hepatocytes that are differentiated from hMSCs without cell fusion using this novel culture system display the phenotypic and biochemical characteristics and functional activity of normal mature hepatocytes.

### EXAMPLE 4

### P450 activity assay

Cytochromes P450 (CYP) from families CYP1, CYP2, and CYP3 play a prominent part in the oxidative metabolism of domestic and industrial combustion, and procarcinogens (W. F. Busby, J. M. Ackermann, C. L. Crespi, Drag Metab. Dispos. 27,246,1999). CYP3A4 is the major CYP protein expressed in the adult liver, where it may represent up to 60% of total CYP proteins (T. Shimada, H. Yamazaki, M. Miura, Y Inui, F. P. Guengerich, J. Pharmacol. Exp. Ther. 270, 414, 1994). The present inventors investigated the inducible activation of CYPs in response to inducers (Rif: rifampicin and 3-MC: 3-methylcholanthrene) in hMSC-derived hepatocytes (N. Chauret, A. Gauthier, D. A. Nicoll-Griffith, Drug Metab. Dispos. 26, 1, 1998) (Fig. 3).

P450 activity was measured with the P450-GLO Assay (Promega). Human MSC-derived hepatocytes , hMSCs and primary human hepatocytes were treated with a number of CYP inducers for 2 days. The effects of 3-methylcholanthrene were studied at 1 mM, and rifanpicin and dexamethasone at 50 mM. All inducers were dissolved in DMSO, resulting in a final vehicle concentration of 0.1 % (v/v). One set of vehicle controls was used for measuring basal P450 activities and a second set for a background luminescence measurement. After 2 days of induction treatment, P450-GLO luminogenic CYP450 substrates were added to each well according to manufacturer's guidelines. After cytochrome P450 reaction and incubated for an additional hour at room temperature, Reconstituted Luciferin Detection Reagent (LDR) was then added to each well and incubated for 20 minutes. Relative Light Units (RLUs) were recorded with a plate reader.

Activity of CYP3A4 in hMSC-derived heaptocytes was increased upon exposure to inducers similarly to primary cultured human hepatocytes (Fig. 3A). The level of CYP3A4 activity under normal culture conditions was approximately two-fold more in hMSC-derived hepatocytes in the Rif-containing medium. Furthermore, the CYP3A4 expression level was restored to normal levels in the culture medium without inducers after induction (data not shown). The results revealed similar concentrations of CYP3A4 in hMSC-derived hepatocytes (0.13±0.029 pmol) and primary human hepatocytes (0.13±0.014 pmol).

Levels of CYP1A1 and CYP2C9 activities were both increased by inducers (Fig. 3, B and C). On the other hand, CYP activity was not detected in untreated-hMSCs using several condition media. These results suggest that hMSC-derived hepatocytes used in an adherent monolayer culture format can facilitate screening of new chemical entities for induction of human P450 enzymes in a timely and efficient manner.

### EXAMPLE 5

### RT-PCR analysis of MRP gene expression in hMSC-derived hepatocytes

Like CYP3A4, the P-glycoprotein (P-gp) transporter is expressed in apical enterocytes and hepatocytes. The P-gp acts to pump drugs back into the intestinal lumen directly from the gut wall and indirectly from the liver via bile, thus reducing the bioavailability of orally administered drugs (J. H. Lin, M. Yamazaki, Clin. Pharmacokinet. 42, 59, 2003). Multidrug resistance-associated proteins (MRP) from the families MRP1, MRP2, and MRP3 are major liver drug transporters mediating biliary secretion of organic anions such as P-gp (L, Vernhet, M. P. Seite, N. Allain, A. Guillouzo, O. Fardel, J. Pharmacol. Exp. Ther. 298, 234, 2001).

Therefore, the present inventors ascertained the MRP genes expression profile of hMSC-derived hepatocytes by RT-PCR (Fig. 4A). Additionally, humans have two types of known P-gp genes: multidrug resistant protein 1 (MDR1) and MDR3 without MRPs (C. Chen et al., J. Biol. Chem. 265, 506, 1990; C. R. Lincke, J. J. Smit, T. Van der Velde-Koerts, P. Borst. J. Biol. Chem. 266, 53 03, 1991).

Primers used for RT-PCR are described below.
Human MRP1
   F: 5'-cgtacttgaactggctggtt-3' (SEQ ID NO: 1)
   R: 5'-tccagacttcttcatccgag-3' (SEQ ID NO: 2)
Human MRP2
   F: 5'-tcacttgtgacatcggtagc-3' (SEQ ID NO: 3)
   R: 5'-atcttcccgttgtctaggac-3' (SEQ ID NO: 4)
Human MRP3
   F: 5'-actgtggagctcagtgtgtt-3' (SEQ ID NO: 5)
   R: 5'-ggcatccaccttagtatcac-3' (SEQ ID NO: 6)
Human MDR1
   F: 5'-acataaactcatgagctttgag-3' (SEQ ID NO: 7)
   R: 5"-cacgagctatggcaatgcgt-3' (SEQ ID NO: 8)
Human MDR3
   F: 5'-cgatttggtgcatatctcattgtga-3' (SEQ ID NO: 9)
   R: 5'-cccttatctcccactcttgtttc-3' (SEQ ID NO: 10)
Human CD81
   F: 5'-acactgactgctttgaccac-3' (SEQ ID NO: 11)
   R: 5'-agcaccatgctcaggatcat-3' (SEQ ID NO: 12)
Human β-actine
   F: 5'- agagcaagag aggtatcctg-3' (SEQ ID NO: 13)
   R: 5'- agagcatagc cctcgtagat-3' (SEQ ID NO: 14)
   RT-PCR analysis showed that gene expressions of MDR1 and MDR3 were detected to hMSC-derived hepatocytes (Fig. 4B). The levels of all MRPs in hMSC-derived hepatocytes were comparable to those levels in primary human hepatocytes. Thus, hepatocytes differentiated from hMSCs displayed an expression pattern and mRNA levels similar to those for primary human hepatocytes.

### EXAMPLE 6

### Hepatitis C virus (HCV) infection

No high efficient *in-vitro* system for the infection of hepatitis C virus (HCV) is so far available, and thus it remains to clarify the mechanisms of the infection of HCV, the process of viral replication, and the release of viral particles from cells.

There are two types of conventional approaches to infect cultured cells with HCV. One approach is to infect cultured cells derived from the liver with HCVto. The other approach is to infect cells derived from lymphocytes with HCV based on the fact that Flavivirus propagates in lymphocyte lineage cells. It has been reported that HCV can infect and propagate in Molt4-Ma cells, HPB-Ma cells, and MT-2 cells, which are all lymphocyte lineage cells. This suggests that some of HCV that infects human can be lymphotropic viral strains.

On the other hand, HCV infects cultured cells derived from hepatocarcinoma, but hardly propagates in the cells. It has been reported that HCV infects primary culture cells of human fetal and chimpanzee liver and replicates in the cells. Furthermore, it has also been reported that HCV infects PH5CH8 cells derived from primary hepatocytes immortalized with SV40 large T antigen and propagates in the cells, and viral particles are released in the culture supernatant. However, the viral particles released in the culture supernatant were not infectious to freshly prepared PH5CH8 cells, and thus could not be studied by reverse genetics. The present inventors established IMY cell line by fusing HepG2 cell, a cultured cell line derived from hepatocarcinoma, with primary culture hepatocytes derived from human. The inventors have reported that IMY cells are susceptible to HCV infection and viral particles released from IMY cells in the culture supernatant are infectious to freshly prepared IMY cells. Thus, the cell line makes it possible to achieve reverse genetics for the virus (T. Ito et. al., Acquisition of Susceptibility to Hepatitis C Virus Replication in HepG2 Cells by Fusion with Primary Human Hepatocytes: Establishment of a Quantitative Assay for HCV Infectivity in a Cell Culture System. Hepatology 34:566-572, 2001). However, the viral titer after infection and propagation in cells of established hepatic cell line is markedly lower than that in primary culture cells derived from human liver. Thus, it has been desirable to establish a cell line which is substituted for primary culture cells derived from human liver.

Thus, the present inventors attempted to establish a culture system that ensures high efficient HCV infection using hepatocytes differentiated from mesenchymal stem cells.

Hepatocytes differentiated from mesenchymal stem cells were plated in 12-well and 6-well cell culture plates coated with collagen or matrigel. After adhering to the plates, the cells were washed once with Williams E culture medium. Aliquots of a serum obtained from a subject infected with HCV, which had been confirmed to contain infectious HCV particles, were inoculated to the cells. The titer of HCV inoculated was 0.5 or 1.0 HCV gene copy/cell. The plates were incubated in a CO₂ incubator at 37 °C for 3 hours to allow the cells to adsorb the viral particles, and then the cells were washed three times with Williams E culture medium to remove unadsorbed HCV. Aliquots of the hepatocyte culture were added to the plates. The cells were incubated in a CO₂ incubator at 37°C. Aliquots of the cells were sampled daily over 10 days stating from the next day after the initiation of viral culture. The cell sampling was carried out by removing the culture medium from each well and adding 5 M guanidine hydrochloride to the wells. The cell lysates prepared using guanidine hydrochloride were stored at -80°C until HCV RNA was extracted. RNA was extracted according to a conventional method from the cell lysates prepared using guanidine hydrochloride. The HCV RNA was quantitated by real time PCR. The quantitation of HCV RNA was carried out by the method described in (T. Takeuch. et al., Real-Time Detection System for Quantification of Hepatitis C Virus Genome. Gastroenterology, 1999, 116:636-642).

The result showed that the efficiency of viral infection was about 100-1000 times higher when HCV infected hepatocytes differentiated from mesenchymal stem cells than that when the virus infected IMY cells, which had been established by fusing HepG2 cell derived from hepatocarcinoma with primary culture hepatocytes derived from human.

It was recently reported that bone marrow-derived stem cells can repair damaged liver by cell fusion within the host liver and not by converting directly into hepatic cells (G. Vassilopoulos, P. R. Wang, D. W. Russell, Nature. 422, 823, 2003; X. Wang et al., Nature. 422, 897,2003). Induced fusion of cells may conceivably achieve the rescue of damaged liver tissue, but such a strategy is unlikely since the fusion mechanisms are not fully understood. In contrast, the present inventors' HIFC differentiation system was able to produce hepatocyte-like cells by direct differentiation under monoculture conditions. In addition, the HIFC differentiation system does not require co-culture conditions with fetal or adult hepatocytes. The present inventors investigated the consequences of mixing hMSC-derived hepatocytes with human cultured adult hepatocyes in HCM containing interleukin 3, and plating the mixture on a collagen-coated culture dish. Cell fusion was not observed by the G-banding method. This data suggests that hMSC-derived hepatocytes are unlikely to be generated by cell fusion with host hepatocytes.

The ability of new molecular entities to induce CYPs can be assessed in a variety of ways to predict drug-drug interactions (A. D. Rodrigues, Pharm. Res. 14, 1504, 1997; A. D. Rodrigues, Med. Chem. Res. 8, 422, 1998). One method involving enzyme induction requires the use of animal models. Unfortunately, in many cases, the information obtained from laboratory animals cannot be readily extrapolated to humans because there are critical species differences in the induction of several CYPs (J. L. Barwick et al., Mol. Pharmacol. 50, 10, 1996; H. Shin, G. V. Pickwell, D. K. Guenette, B. Bilir, L. C. Quattrochi, Hum. Exp. Toxicol. 18, 95, 1999).

Human fetal hepatocytes constitute today a standard model system in mature hepatocyte research. Drawbacks include the limited amount of cells that can be obtained from an individual, a limited life span and incapacity to withstand freeze/thaw procedures. Additionally, primary cultures of human hepatocytes can be used to predict enzyme induction (P. Maurel, Adv. Drug Deliv. Rev. 22, 105, 1996; P. Maurel, Adv. Drug Deliv. Rev. 22, 105, 1996; E. LeCluyse et al., J. Biochem. Toxicol. 14,177, 2000). However, there are limitations associated with this system: results obtained from human hepatocyte cultures frequently show marked sample-to-sample variability in response to P450 enzyme inducers, thereby making it important to screen samples from several individuals (E. LeCluyse et al., J. Biochem. Toxicol. 14,177 2000). Other disadvantages of using hepatocytes include a high cost and the need for fresh human livers, which are available sporadically. The present invention reveals, for the first time, that functional hepatocytes displaying CYPs, MRPs and MDRs activity can be induced from hMSCs using direct differentiation conditions *in vitro*. The system of this invention will provide a valuable tool for studying the molecular basis of the developmental processes influencing hepatic cells *in vitro*, screening of candidate new drugs and may form the basis for cell therapies applicable for the rescue of hepatic damage.

## Claims

1. A method for preparing human hepatocyte-like cells, which comprises the steps of:
(a) incubating undifferentiated cells derived from human in a culture medium containing a combination of growth factors selected from (i) to (iii) below:
(i) acidic fibroblast growth factor, fibroblast growth factor 4, and hepatocyte growth factor;
(ii) acidic fibroblast growth factor and a growth factor selected from the group consisting of activin A, epidermal growth factor, and β-nerve growth factor; and
(iii) fibroblast growth factor 4 and a growth factor selected from the group consisting of activin A and hepatocyte growth factor; ;
(b) incubating the cells cultured in step (a) in a culture medium containing oncostatin M; and
(c) incubating the cells cultured in step (b) in a culture medium containing dexamethasone, wherein the total culturing period is about 2 to about 13 weeks.

2. The method according to claim 1, which comprises using a collagen-coated culture dish.

3. The method according to claim 1, wherein the undifferentiated cells derived from human are embryonic stem cells, adult stem cells, mesenchymal stem cells, cord blood cells, or somatic cells with artificially conferred multipotency.

4. Human hepatocyte-like cells prepared by the method according to any one of claims 1 to 3.

5. A therapeutic agent for hepatic diseases, which comprises the human hepatocyte-like cells according to claim 4.

6. A method for assessing the metabolism of a test compound, which comprises the steps of:
(a) contacting a test compound with the human hepatocyte-like cells according to claim 4; and
(b) determining the metabolism of the test compound contacted with the human hepatocyte-like cells.

7. A method for assessing hepatotoxicity of a test compound, which comprises the steps of:
(a) contacting a test compound with the human hepatocyte-like cells according to claim 4; and
(b) determining the degree of damage of the human hepatocyte-like cells contacted with the test compound.

8. A method of screening for a therapeutic agent for hepatic diseases, which comprises the steps of:
(a) contacting a test compound with the human bepatocyte-like cells according to claim 4;
(b) determining the function of the human hepatocyte-like cells contacted with the test compound; and
(c) selecting a compound that enhances the function of the human hepatocyte-like cells contacted with the test compound.

9. A method of screening for an inhibitor to hepatitis virus infection, which comprises the steps of:
(a) contacting the human hepatocyte-like cells according to claim 4 with hepatitis virus in the presence of a test compound;
(b) examining the infection of hepatitis virus to the human hepatocyte-like cells contacted with hepatitis virus; and
(c) selecting a compound that inhibits the infection of hepatitis virus.

10. A method of screening for a therapeutic agent for viral hepatitis, which comprises the steps of:
(a) contacting the human hepatocyte-like cells according to claim 4 with hepatitis virus;
(b) contacting a test compound with the human hepatocyte-like cells infected with hepatitis virus;
(c) determining the degree of hepatitis virus propagation in the cells contacted with the test compound; and
(d) selecting a compound that inhibits the propagation of hepatitis virus.


SEQUENCE LISTING

<110> EFFECTOR CELL INSTITUTE, INC.
OCHIYA, Takahiro

<120> Human hepatocyte-like cells and uses thereof

<130> KAN-X0401

<160> 14

<170> PatentIn version 3.3

<210> 1
<211> 20
<212> DNA
<213> Artificial

<220>
<223> An artificially synthesized primer sequence

<400> 1
cgtacttgaa ctggctggtt 20

<210> 2
<211> 20
<212> DNA
<213> Artificial

<220>
<223> An artificially synthesized primer sequence

<400> 2

tccagacttc ttcatccgag                                                    20

<210>  3
<211>  20
<212>  DNA
<213>  Artificial

<220>
<223>  An artificially synthesized primer sequence

<400>  3
tcacttgtga catcggtagc                                                    20

<210>  4
<211>  20
<212>  DNA
<213>  Artificial

<220>
<223>  An artificially synthesized primer sequence

<400>  4
atcttcccgt tgtctaggac                                                    20

<210>  5
<211>  20
<212>  DNA
<213>  Artificial

<220>
<223>  An artificially synthesized primer sequence

<400> 5
actgtggagc tcagtgtgtt 20

<210> 6
<211> 20
<212> DNA
<213> Artificial

<220>
<223> An artificially synthesized primer sequence

<400> 6
ggcatccacc ttagtatcac 20

<210> 7
<211> 22
<212> DNA
<213> Artificial

<220>
<223> An artificially synthesized primer sequence

<400> 7
acataaactc atgagctttg ag 22

<210> 8
<211> 20
<212> DNA
<213> Artificial

<220>
<223> An artificially synthesized primer sequence

<400> 8
cacgagctat ggcaatgcgt 20

<210> 9
<211> 25
<212> DNA
<213> Artificial

<220>
<223> An artificially synthesized primer sequence

<400> 9
cgatttggtg catatctcat tgtga 25

<210> 10
<211> 23
<212> DNA
<213> Artificial

<220>
<223> An artificially synthesized primer sequence

<400> 10
cccttatctc ccactcttgt ttc 23

<210> 11
<211> 20
<212> DNA
<213> Artificial

<220>

<223> An artificially synthesized primer sequence

<400> 11
acactgactg ctttgaccac 20

<210> 12
<211> 20
<212> DNA
<213> Artificial

<220>
<223> An artificially synthesized primer sequence

<400> 12
agcaccatgc tcaggatcat 20

<210> 13
<211> 20
<212> DNA
<213> Artificial

<220>
<223> An artificially synthesized primer sequence

<400> 13
agagcaagag aggtatcctg 20

<210> 14
<211> 20
<212> DNA
<213> Artificial

<220>
<223> An artificially synthesized primer sequence

<400> 14
agagcatagc cctcgtagat 20